# EUROPEAN PATENT APPLICATION

(11) **EP 4 628 096 A1**
(43) Date of publication of application: **08.10.2025**
(21) Application number: 23895042.2
(22) Date of filing: 23.11.2023
(51) Int. Cl.: A61K 39/135, A61P 31/14

(54) **RECOMBINANT EXPRESSION VECTOR FOR PREPARING FOOT-AND-MOUTH DISEASE VIRUS-LIKE PARTICLES OR NANOPARTICLES, AND VACCINE COMPOSITION USING SAME**

(30) Priority: 23.11.2022 KR 20220158630
(71) Applicant: Optipharm Co., Ltd., Cheongju-si, Chungcheongbuk-do 28158 (KR)
(72) Inventor: LEE, Ji Hoon, Cheongju-si Chungcheongbuk-do 28609 (KR); PARK, Sang Mi, Cheongju-si Chungcheongbuk-do 28315 (KR); OH, Jong Min, Cheongju-si Chungcheongbuk-do 28541 (KR); CHOI, Jae Bang, Cheongju-si Chungcheongbuk-do 28748 (KR); HAN, Beom Ku, Daejeon 35243 (KR); KIM, Hyun Il, Cheongju-si Chungcheongbuk-do 28162 (KR)
(74) Representative: Potter Clarkson
(86) International application number: PCT/KR2023/018972
(87) International publication number: WO 2024/112115

(57) **Abstract**

The present invention relates to a recombinant expression vector for preparing foot-and-mouth disease virus-like particles or nanoparticles, and a vaccine composition using same. The present invention provides virus-like particles or nanoparticles, and a preparation method therefor, the particles or nanoparticles being produced by expressing, simultaneously, VP4 with VPI, VP2 and VP3 or expressing VP1, VP2 and VP3 while excluding VP4, from among FMDV structural proteins. The virus-like particles or nanoparticles, prepared according to the method of the present invention, can be effectively used in the prevention of diseases caused by foot-and-mouth disease viral infection.

## Description

### [Technical Field]

The present disclosure relates to a recombinant expression vector for producing foot-and-mouth disease virus-like particles or nanoparticles, and a vaccine composition using the same.

### [Background Art]

Foot and Mouth Disease Virus (FMDV) is a virus that causes acute and fatal infectious diseases in cloven-hoofed animals, including cattle and pigs. This virus causes various symptoms such as fever, claudication, and blister formation at the time of infection, and can be transmitted through contact or through people, vehicles, or various objects passing through contaminated areas. In addition, the virus is highly contagious and can spread through the air up to 250 km. As a result, the virus has a serious effect on the livestock industry, resulting in economic losses such as reproductive issues and reduced milk production. Foot-and-mouth disease is a disease designated as a Class 1 livestock infectious disease under the Animal Infectious Disease Control Act by the World Organization for Animal Health (WOAH).

The FMDV is a single-stranded positive-sense RNA virus belonging to the genus Aphtovirus in the family Picornaviridae. Currently, a total of seven serotypes are known as O, A, Asia1, C, SAT1, SAT2, and SAT3, and each serotype is known to have several topotypes. Globally, the O type mainly occurs continuously, and in the East Asian region, such as China and North Korea, Asia1 and A and O types tend to occur intermittently. The FMDV differs genetically and antigenically from viruses of different serotypes, which are not serologically neutralized nor cross-protected by vaccines.

The FMDV has an exposed capsid, which has an icosahedral structure. The P1 region of the FMDV polyprotein encodes structural proteins, and the P2 and P3 regions encode nonstructural proteins. The structural protein precursor P1 is cleaved by viral protease 2A, and the P1 precursor is processed into capsid proteins VP0, VP3, and VP1. 3C is a viral protease that serves to process the P1 precursor into a capsid protein. In a virion, the protein VP0 is cleaved into two proteins, VP4 and VP2.

Inactivated virus vaccines are commercially available and sold to prevent foot-and-mouth disease. Generally, the virus is cultured in cells, inactivated with ethyleneimine, and then mixed with an adjuvant to be used as a vaccine. Current foot-and-mouth disease vaccines have been indicated to have disadvantages of short antibody persistence and low immunogenicity in pigs, and thus there is a need to develop more effective and stable vaccines. In particular, when intramuscularly administered into pigs, there are disadvantages such as local side effects, including lesion formation in the injected muscle, fibrosis, granuloma at the injection site, and low safety. The foot-and-mouth disease vaccine, which was administered intramuscularly with a commercial vaccine, caused abnormal meat problems by remaining in the form of granulomas or abscesses until the time of shipment, and the abnormal meat became a main reason why farms that suffered economic losses avoided vaccination. An ideal vaccine design is needed to overcome these limitations of current commercial vaccines.

Accordingly, the present inventors prepared virus-like particles or nanoparticles by simultaneously expressing VP4 and VP1, VP2, and VP3 among FMDV structural proteins, or by expressing VP1, VP2, and VP3 excluding VP4, and confirmed that a vaccine composition using these may induce effective immune responses, thus completing the present disclosure.

### [Disclosure]

### [Technical Problem]

An object of the present disclosure is to provide a recombinant expression vector including an FMDV antigen, a transformant transformed with the recombinant expression vector, virus-like particles or nanoparticles prepared by the transformant, and a method for preparing the same.

Another object of the present disclosure is to provide a vaccine composition for an FMDV and a method for preventing diseases caused by FMDV infection.

Yet another object of the present disclosure is to provide a use of the virus-like particles or nanoparticles for the prevention of FMDV diseases.

### [Technical Solution]

To achieve the above object, an aspect of the present disclosure provides a recombinant expression vector including a first promoter and genes encoding Foot and Mouth Disease Virus (FMDV) proteins VP2, VP3, VP1 and 3C operably linked to the promoter.

Another aspect of the present disclosure provides a recombinant vector including a first promoter and genes encoding FMDV VP2, VP3, VP1 and 3C operably linked to the promoter; and a second promoter and a gene encoding a FMDV protein VP4 operably linked to the promoter.

Yet another aspect of the present disclosure provides a transformant transformed with the recombinant expression vector, virus-like particles or nanoparticles prepared by the transformant, and a method for preparing the same.

Still another aspect of the present disclosure provides a vaccine composition for a FMDV including the virus-like particles or nanoparticles as an active ingredient.

Still another aspect of the present disclosure provides a method for preventing diseases caused by FMDV infection, including administering the vaccine composition.

Still another aspect of the present disclosure provides a use of the virus-like particles or nanoparticles for preventing FMDV diseases.

### [Advantageous Effects]

The present disclosure relates to a recombinant expression vector for preparing foot-and-mouth disease virus-like particles or nanoparticles and a vaccine composition using the same. The present disclosure provides virus-like particles or nanoparticles, and a preparation method therefor, the particles or nanoparticles being produced by expressing, simultaneously, VP4 with VP1, VP2 and VP3 or expressing VP1, VP2 and VP3 while excluding VP4, among FMDV structural proteins. The virus-like particles or nanoparticles, prepared according to the method of the present disclosure, can be effectively used in the prevention of diseases caused by foot-and-mouth disease viral infection.

### [Description of Drawings]

FIG. 1 is a schematic diagram showing a VP4 Co-expression form of the present disclosure.
FIG. 2 is a schematic diagram showing a Delta-VP4 expression form of the present disclosure.
FIG. 3 is a schematic diagram showing a full form.
FIG. 4 is a diagram showing results of confirming through Coomassie staining whether a recombinant virus of the present disclosure expresses target proteins.
FIG. 5 is a diagram showing results of confirming through Western blot whether a recombinant virus of the present disclosure expresses a VP1 protein.
FIG. 6 is a diagram showing results of confirming the expression of the VP1 protein using a culture medium cultured with a recombinant virus transformed into a Delta-VP4 form.
FIG. 7 is a diagram showing results of confirming through Coomassie staining whether a recombinant virus transformed into a Full form expresses target proteins.
FIG. 8 is a diagram showing results of confirming the expression of the VP1 protein using a culture medium cultured with a recombinant virus transformed into a Full form.
FIG. 9 is a diagram showing results of comparing antigen amounts by performing ELISA using antibodies specific for an FMDV VP1 protein.
FIG. 10 is a diagram showing results of confirming the expression of an O type FMDV VP1 protein through Western blot.
FIG. 11 is a diagram showing results of confirming the expression of an O type FMDV VP2 protein through Western blot.
FIG. 12 is a diagram showing results of confirming the expression of an O type FMDV VP3 protein through Western blot.
FIG. 13 is a diagram showing results of confirming the expression of an O type FMDV VP4 protein through Western blot.
FIG. 14 is a diagram showing results of confirming the expression of an A type FMDV VP1 protein through Western blot.
FIG. 15 is a diagram showing results of confirming the expression of an A type FMDV VP2 protein through Western blot.
FIG. 16 is a diagram showing results of confirming the expression of an A type FMDV VP3 protein through Western blot.
FIG. 17 is a diagram showing results of confirming the expression of an A type FMDV VP0 protein through Western blot.
FIG. 18 is a diagram showing results of confirming the expression of an A type FMDV VP4 protein through Western blot.
FIG. 19 is a diagram showing nanoparticles of an FMDV antigen candidate observed through TEM.
FIG. 20 is a diagram showing VLP particles of an FMDV antigen candidate observed through TEM.
FIG. 21 is a diagram showing a method for administering a vaccine composition of the present disclosure.
FIG. 22 is a graph showing results of confirming the immunogenicity of vaccine candidates.
FIG. 23 is a graph showing results of measuring antibody titers according to concentrations of vaccine candidates for O and A types.
FIG. 24 is a graph showing results of measuring neutralizing antibody titers according to concentrations of vaccine candidates for O and A types.
FIG. 25 is a diagram showing results of histopathological observation of pigs administered with a vaccine composition prepared from a recombinant virus transformed into a VP4 Co expression form.
FIG. 26 is a diagram showing histopathological observation results of pigs administered with a control vaccine.

### [Best Mode]

Hereinafter, the present disclosure will be described in detail.

The present disclosure provides a recombinant expression vector including a first promoter and genes encoding Foot and Mouth Disease Virus (FMDV) proteins VP2, VP3, VP1 and 3C operably linked to the promoter.

In addition, the present disclosure provides a recombinant vector including a first promoter and genes encoding FMDV VP2, VP3, VP1 and 3C operably linked to the promoter; and a second promoter and a gene encoding a FMDV protein VP4 operably linked to the promoter.

As used herein, the "foot-and-mouth disease virus (hereinafter referred to as FMDV)" belongs to the genus Aphtovirus of the family Picornaviridae. The virus consists of 60 copies of four capsid proteins VP1, VP2, VP3, and VP4 and a single-stranded RNA genome (approximately 8.5 kb), and is a highly contagious disease that infects cloven-hoofed animals, especially cattle, pigs, and sheep.

The four capsid proteins of FMDV are VP1, VP2, VP3, and VP4, and among them, VP1, VP2, and VP3 are proteins exposed on the capsid surface, VP1 is most associated with the infectivity of the virus, and VP4 is a small protein and located therein. There are many serotypes depending on a region, there are 7 serotypes including A, O, C, SAT1, SAT2, SAT3, and Asia1 depending on an antigen structure, and these serotypes have more than 80 serosubtypes.

As used herein, the "Virus-Like Particle (VLP) and Nanoparticle" are antigens having almost the same shape as a virus, including nonstructural proteins that are responsible for replication and intracellular penetration of the virus, and has almost the same shape as a virus by combining structural surface (envelope) proteins representing the antigenicity of the virus using genetic recombination technology without including a genetic material.

The envelop region of the FMDV is an antigenic region most reliably detected by an immune system after in vivo infection, and it is possible to prepare a safe and effective new vaccine using a protein that forms the envelop.

The recombinant expression vector includes a polynucleotide in which base sequences of VP2, VP3, VP1, and 3C of the FMDV are arranged sequentially, and when expressed as a protein, it is possible to prepare FMDV-like particles by self-assembly. In addition, the recombinant expression vector may be prepared to express a base sequence of VP4 in an opposite direction to the polynucleotide.

In the present disclosure, the FMDV may be selected from O, A, Asia1, C, SAT1, SAT2, and SAT3 types.

In addition, the FMDV O type may be selected from the group consisting of O-Andong, O-PanAsia2 (O-PA2), O-manisa, O-Taiwan97 (O-Twn97), O-Campos, O-Boeun (O-BE), O-Jincheon (O-JC), O-Anseong (O-AS), and O-Gimje (O-GJ). In some embodiments of the present disclosure, the O type may use a gene sequence of O-Andong (GenBank: KF112887.1). In addition, in some embodiments, to improve the expression level of the protein, the nucleic acid sequence was codon-optimized using an insect cell preferred codon in the present disclosure. As an example, the base sequences encoding the VP1, VP2, VP3, VP4 and 3C proteins may be base sequences as set forth in SEQ ID NO: 1, SEQ ID NO: 2, SEQ ID NO: 3, SEQ ID NO: 4 and SEQ ID NO: 9, respectively, or variants thereof. In addition, the O type VP1, VP2, VP3, or VP4 protein may consist of an amino acid sequence as set forth in any one of SEQ ID NO: 10, SEQ ID NO: 11, SEQ ID NO: 12, and SEQ ID NO: 13, respectively, or may include a variant thereof.

In addition, the FMDV A type may be selected from the group consisting of A-Pocheon (A-PC), A-Yeoncheon (A-YC), A-Bangladesh (A-Ban), A-Malaysia97 (A-May97), A-Gimpo (A-GP), and A22-Iraq. In some embodiments of the present disclosure, the A type may use a gene sequence of A-Pocheon (GenBank: KC588943.1) or A-Yeoncheon (GenBank: KY766148.1). In addition, in some embodiments, in order to improve the expression level of the protein, the nucleic acid sequence was codon-optimized using an insect cell preferred codon in the present disclosure. As an example, the base sequences encoding the VP1, VP2, VP3, VP4 and 3C proteins may be base sequences as set forth in SEQ ID NO: 5, SEQ ID NO: 6, SEQ ID NO: 7, SEQ ID NO: 8 and SEQ ID NO: 9, respectively, or variants thereof. In addition, the A type VP1, VP2, VP3, or VP4 protein may consist of an amino acid sequence as set forth in any one of SEQ ID NO: 14, SEQ ID NO: 15, SEQ ID NO: 16, and SEQ ID NO: 17, respectively, or may include a variant thereof.

The 3C may be a base sequence encoding an amino acid of the base sequence as set forth in SEQ ID NO: 9 or a variant thereof, and may consist of an amino acid sequence as set forth in SEQ ID NO: 18 or include a variant thereof. The FMDV 3C is a protease and cleaves FMDV VP4, VP2, VP3, and VP1 proteins to enable self-assembly of FMDV VLP.

The 3C has a common conserved sequence that does not differ in sequence by FMDV serotype. However, the sequence of VP4-VP2-VP3-VP1 of FMDV is different in serotypes O, A, Asia1, SAT, and C, and even in the same serotype, the sequences are different by serosubtype.

In the present disclosure, the recombinant expression vector may further include any sequences, preferably a promoter and/or a sequence (e.g., 2A sequence and/or Burst sequence, etc.) for promoting expression, as long as the recombinant expression vector may increase the expression levels or efficiency of target proteins.

As used herein, the "promoter" may be applied without limitation as long as the promoter is a sequence sufficient to induce transcription. As an embodiment, a promoter (polyhedrin promoter) of a polyhedrin protein coding gene is located in front of a target protein of VP2-VP3-VP1-3C to increase the expression efficiency of the target protein. The promoter of the polyhedrin protein coding gene may be a base sequence as set forth in SEQ ID NO: 19 or a variant thereof. In addition, the promoter of the polyhedrin protein coding gene may be located in front of a vp39 promoter (SEQ ID NO: 20). Meanwhile, different promoters may be used for the expression of the target protein of VP4. As an embodiment, a p10 promoter may be located in front of the target protein of VP4 to increase the expression efficiency of the target protein, and the p10 promoter may be a base sequence as set forth in SEQ ID NO: 21 or a variant thereof.

The recombinant expression vector may further include a 2A sequence. The 2A gene sequence encodes 18 to 22 amino acids, and among them, four amino acids located at the end, Asparagine (N), Proline (P), Glycine (G), and Proline (P), are amino acids that are significantly conserved among species. When synthesized with peptides, the sequence shows a tendency for self-cleavage. Due to such a property, when a ribosome reaches a genetic code encoding N, P, and G located at the end of the 2A sequence during protein transcription, NPG are recognized in sequence to form a peptide bond and then bring a releasing factor RF instead of bringing prolyl-tRNA with Proline bound to the next amino acid, a proline encoding code. The RF factor binds, and then the previously formed peptides no longer form the peptide bonds and are released from the ribosome. In addition, after the 2A sequence, the encoded code operates normally to perform the next protein transcription. In conclusion, many genes may be expressed using a single promoter by inserting the 2A sequence. The recombinant expression vector of the present disclosure may simultaneously express the genes by inserting the 2A sequence between the genes. In one embodiment, when the base sequences encoding the VP2-VP3-VP1-3C protein are arranged sequentially, the 2A sequence may be further included between the VP1 and 3C genes. In one embodiment, the 2A sequence may be a base sequence as set forth in SEQ ID NO: 22 or SEQ ID NO: 23, or a variant thereof.

The recombinant expression vector may further include a Burst sequence. The Burst sequence is a portion present in the polyhedrin promoter and a sequence located between a translation initiation site and TAAG, and is known that Vlf-1 specifically binds to the Burst sequence at the end of baculovirus infection to promote transcription. In one embodiment, the Burst sequence may be a base sequence as set forth in SEQ ID NO: 24 or a variant thereof.

As used herein, the "vector" refers to any medium for cloning and/or transferring bases into a host cell. The vector may be a replicating unit (replicon) to which other DNA fragments may be bound to bring replication of the bound fragments. The "replicon" is any genetic unit (e.g., plasmid, phage, cosmid, chromosome, and virus) that functions as an autonomous unit of DNA replication in the body, i.e., is replicable by self-regulation. The term "vector" includes viral and non-viral mediums s for introducing bases into a host cell in vitro, ex vivo or in vivo.

As used herein, the term "recombinant expression vector" is a vector prepared so as to express a target protein in suitable host cells and refers to a gene construct including a required regulatory element which is operably linked so that a gene insert is expressed. The recombinant expression vector of the present disclosure may include genes encoding FMDV proteins VP2, VP3, VP1 and 3C or include a gene encoding a FMDV protein VP4. At this time, the genes encoding the FMDV proteins VP2, VP3, VP1 and 3C and the gene encoding the FMDV protein VP4 may be linked to two different promoters in one vector, respectively, and the two promoters in the vector are positioned in opposite directions to minimize an effect between the two promoters.

In the present disclosure, the term "variant" as used herein for a nucleic acid may mean (i) a portion or fragment of a referenced nucleotide sequence; (ii) a complement of a referenced nucleotide sequence or a portion thereof; (iii) a nucleic acid substantially identical to a referenced nucleic acid or a complement thereof; or (iv) a nucleic acid that hybridizes under stringent conditions to a referenced nucleic acid, a complement thereof, or a sequence substantially identical thereto.

As used herein, the "variant" for a peptide or polypeptide has a different amino acid sequence by insertion, deletion, or conservative substitution of amino acids, but maintains at least one biological activity. The variant may also mean a protein having an amino acid sequence substantially identical to a referenced protein having amino acids with at least one biological activity. Conservative substitutions of amino acids, i.e., replacement of amino acids using different amino acids having similar properties (e.g., hydrophilicity, degree and distribution of charged regions), are recognized in the art to typically include small changes.

In addition, the present disclosure provides a transformant transformed with the recombinant expression vector.

In the present disclosure, the description of the "recombinant expression vector" is as described above.

In the present disclosure, the "transformation" means a phenomenon in which DNA is introduced into a host to enable the DNA to replicate as a chromosomal factor or by chromosomal integration, and external DNA is introduced into a cell to artificially cause a genetic change.

The term "transformant" as used herein means a transgenic plant or transgenic animal produced by transformation, and includes a genetic recombinant produced by inducing a modification or mutation of a specific gene using genetic recombination technology.

In the present disclosure, the host cell for preparing the "transformant" is preferably a host cell having high introduction efficiency of DNA and high expression efficiency of the introduced DNA, and may be used with all microorganisms including prokaryotes and eukaryotes. The host cell may be selected from the group consisting of Escherichia sp. bacteria; Bacillus sp. bacteria; Pseudomonas sp. bacteria; lactic acid bacteria; yeast; animal cells; and insect cells, and preferably, insect cells.

The transformant is for preparing foot-and-mouth disease virus-like particles or nanoparticles.

In addition, the present disclosure provides virus-like particles or nanoparticles prepared by the transformant.

In the present disclosure, the descriptions of "transformation" and "virus-like particle or nanoparticle" are as described above.

In addition, the present disclosure provides a method for preparing virus-like particles or nanoparticles for preventing diseases caused by FMDV infection, including: (1) preparing a recombinant expression vector of the present disclosure; (2) preparing a transformant using the recombinant expression vector; (3) transfecting the transformant into a host cell; (4) culturing the transfected host cell and collecting a culture medium thereof; and (5) isolating virus-like particles or nanoparticles from the culture medium.

In the present disclosure, the descriptions of the "recombinant expression vector", "host cell", "transformant", "foot-and-mouth disease", and "virus-like particles or nanoparticles" are as described above.

Further, the present disclosure provides a vaccine composition for a FMDV including the virus-like particles or nanoparticles as an active ingredient.

As used herein, the term "vaccine" refers to an immunogen or antigenic substance that provides immunity to a living organism by being injected or orally administered to a human or animal to prevent infection, as a biological preparation containing an antigen that provides immunity to a living organism.

The content of the antigen may be 1 to 15 wt% with respect to the total weight of the vaccine composition.

The vaccine composition of the present disclosure may further include one or more adjuvants.

In the present disclosure, the "adjuvant" generally refers to any substance that increases humoral and/or cellular immune responses to an antigen. Traditional vaccines are composed of unprocessed preparations of killed pathogenic microorganisms, and impurities related to a culture medium of the pathogenic microorganisms may act as adjuvants to enhance the immune responses. However, when a homogeneous preparation of purified protein subunits is used as an antigen for vaccination, the immunity induced by such an antigen is insufficient, and thus it is necessary to add some foreign substances as adjuvants. By using the adjuvant, a smaller dose of antigen may be required to stimulate an immune response, thereby reducing the cost of vaccine production. In some embodiments, the adjuvant includes EMULSIGEN, aluminum hydroxide, Carbigen, saponin, CpG or a combination thereof. In another embodiment, the adjuvant may be a well-known oil emulsion, preferably a single oil emulsion.

The vaccine composition according to the present disclosure may further include at least one second adjuvant selected from the group consisting of a stabilizer, an emulsifier, aluminum hydroxide, aluminum phosphate, a pH adjuster, a surfactant, a liposome, an iscom adjuvant, synthetic glycopeptide, an extender, carboxypolymethylene, a bacterial cell wall, a derivative of the bacterial cell wall, a bacterial vaccine, an animal poxvirus protein, a subviral particle adjuvant, cholera toxin, N,N-dioctadecyl-N',N'-bis(2-hydroxyethyl)-propanediamine, monophosphoryl lipid A, dimethyldioctadecyl-ammonium bromide, and mixtures thereof.

In addition, the vaccine composition of the present disclosure may include a veterinarily acceptable carrier. The term "veterinarily acceptable carrier" as used herein includes any and all solvents, dispersion media, coatings, adjuvants, stabilizers, diluents, preservatives, antibacterial and antifungal agents, isotonic agents, adsorption delaying agents, and the like. The carrier, the excipient, and the diluent that may be included in the vaccine composition may include lactose, dextrose, sucrose, sorbitol, mannitol, xylitol, erythritol, maltitol, starch, glycerin, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

In addition, the vaccine composition of the present disclosure may be formulated and used in the form of oral formulations such as powders, granules, tablets, capsules, suspensions, emulsions, syrups, aerosols, etc., and sterile injectable solutions according to a general method. When the vaccine composition is formulated, the formulations may be prepared by using diluents or excipients, such as a filler, an extender, a binder, a wetting agent, a disintegrating agent, a surfactant, etc., which are generally used. Solid formulations for oral administration include tablets, pills, powders, granules, capsules, and the like, and these solid formulations may be prepared by mixing the lecithin-like emulsifier with at least one or more excipients, for example, starch, calcium carbonate, sucrose or lactose, gelatin, and the like. Further, lubricants such as magnesium stearate and talc may be used in addition to simple excipients. Liquid formulations for oral administration may be used with a suspension, an oral liquid, an emulsion, a syrup, and the like, and may include various excipients, for example, a wetting agent, a sweetener, an aromatic agent, a preserving agent, and the like, in addition to water and liquid paraffin which are commonly used as simple diluents. Formulations for parenteral administration include a sterile aqueous solution, a non-aqueous solution, a suspension, an emulsion, and a lyophilizing agent. As the non-aqueous solution and the suspension, propylene glycol, polyethylene glycol, vegetable oil such as olive oil, injectable ester such as ethyl oleate, and the like may be used.

VLP vaccines express one or more structural proteins of a virus through molecular biology technology, and since these structural proteins have natural self-assembly ability, the structural proteins may form spatial structures and epitopes similar to natural virus particles, but have no viral nucleic acids, are highly immunogenic, and have no infectiousness. In addition, since a high density of viral antigens are present on the surface, the virus may be delivered to immune cells in the same manner as infecting a living organism to effectively induce humoral immunity and cellular immunity of an in vivo immune system and shorten the latent period.

Further, the present disclosure provides a method for preventing diseases caused by FMDV infection, including administering the vaccine composition to a mammal other than a human.

In the present disclosure, the description of the "vaccine composition" is as described above.

As used herein, the "prevention" refers to all actions that inhibit or delay the FMDV infection by administering a composition containing as an active ingredient a FMDV-like particle or nanoparticle protein that self-assembles using the recombinant expression vector of the present disclosure.

In the present disclosure, the mammal may be applied without limitation to animals, such as chickens, pigs, monkeys, dogs, cats, rabbits, guinea pigs, rats, mice, cows, sheep, goats, etc. Preferably, the mammal is a cloven-hoofed animal such as pigs or cattle.

In the present disclosure, the administration may be performed by any administration means known in the art. For example, the vaccine composition may be administered directly to a subject intravenously, intramuscularly, orally, transdermally, mucosally, intranasally, intratracheally, or subcutaneously. The vaccine composition may be administered systemically or locally.

In the present disclosure, the composition of the present disclosure may be administered in a therapeutically or prophylactically effective amount. The "therapeutically or prophylactically effective amount" may be appropriately selected by those skilled in the art by considering the severity of symptoms, the sex, age, and weight of a subject, etc. The therapeutically or prophylactically effective amount may be, for example, 1 pg to 5 g of a FMDV-like particle or nanoparticle self-assembled using the polynucleotide, a protein extract of the transformant, or a recombinant protein isolated from the transformant, per 1 kg of the administered subject.

Further, the present disclosure provides a use of the virus-like particles or nanoparticles for preventing FMDV diseases.

In the present disclosure, the descriptions of "FMDV" and "virus-like particle or nanoparticle" are as described above.

### [Modes]

Hereinafter, Examples of the present disclosure will be described in detail with reference to the accompanying drawings so as to be easily implemented by those with ordinary skill in the art to which the present disclosure pertains. However, the present disclosure may be implemented in various different forms and is not limited to Examples described herein.

### <Example 1> Preparation of recombinant vectors and viruses expressing FMDV antigen candidates

To use a high-expression baculovirus expression system, a transfer vector capable of expressing structural proteins and major antigen candidates of FMDV was prepared.

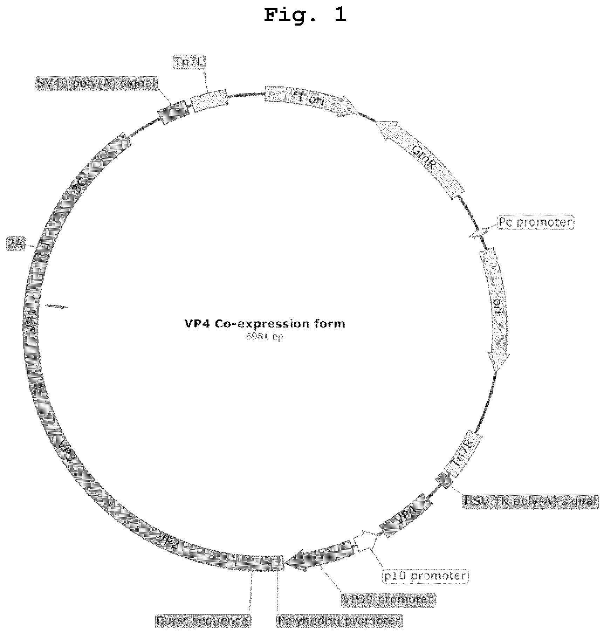

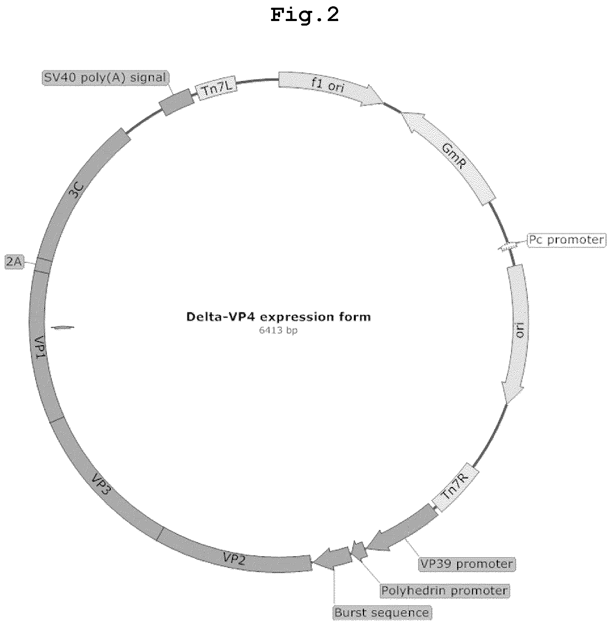

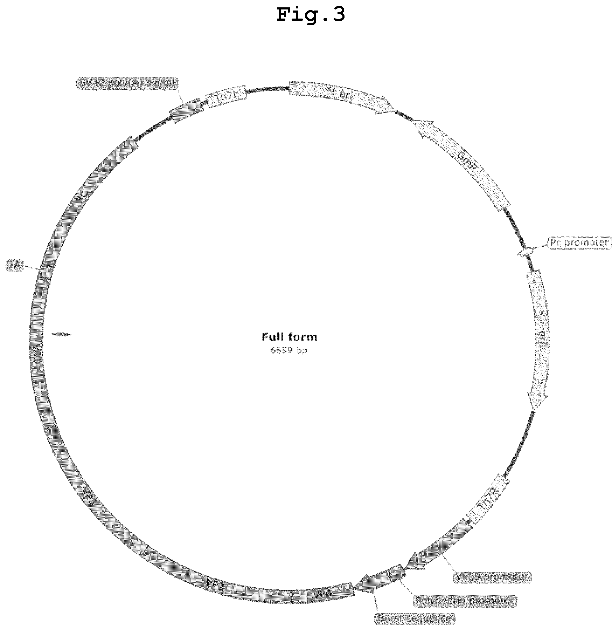

Specifically, a Delta-VP4 expression form was prepared by cloning target genes listed in the order of VP2, VP3, VP1, and 3C behind a polyhedrin promoter using a high-expression vector pPol-6 (FIG. 1). Thereafter, a p10 promoter and VP4 were cloned in the opposite direction to the Polyhedrin promoter of Delta-VP4 expression to prepare a VP4 Co-expression form (FIG. 2). Meanwhile, as a control group, a full form was prepared by cloning target genes listed in the order of VP4, VP2, VP3, VP1, and 3C behind the Polyhedrin promoter (FIG. 3). To improve cloning efficiency, codon-optimized gene sequences were inserted into the vector. A recombinant transfer vector including a codon-optimized base sequence and an O type epitope corresponding to an amino acid sequence was prepared. In addition, a recombinant transfer vector including a codon-optimized base sequence and an A type epitope corresponding to an amino acid sequence was prepared.

Each of the prepared transfer vectors was inserted into Bacmid, in which chitinase and chathepsin are removed from a virus based on AcMNPV (Autographa californica multiple nucleopolyhedrovirus), to prepare a recombinant virus, and then the recombinant virus was proliferated in Sf9 cells, an insect cell line, to be mass-produced.

### <Example 2> Identification of proteins expressed from recombinant virus expressing FMDV structural proteins

### <2-1> Results of electrophoresis analysis of proteins expressed from recombinant virus expressing FMDV structural proteins

An experiment was performed to confirm whether the recombinant virus expressing the FMDV structural proteins prepared in Example 1 expressed target proteins.

Specifically, the recombinant virus was inoculated into insect cells Hi5, and after several days, the cells and culture medium were collected by centrifugation at 4000 rpm, 5 minutes, and 4°C. The collected cells were treated with an equal volume of PBS as the culture medium and resuspended. The collected cells and culture medium samples were subjected to SDS-PAGE and Western blotting, respectively.

First, proteins expressed from recombinant viruses transformed into the Delta-VP4 form and a VP4 Co-expression form were analyzed.

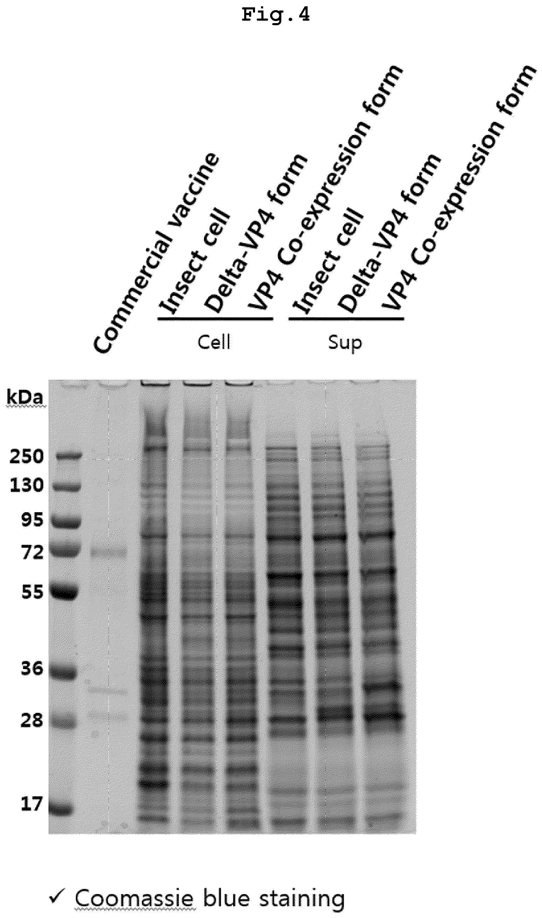

As a result, as shown in FIG. 4, clear bands were identified at target protein positions of 23.1 kDa corresponding to a VP2 protein, 24.4 kDa corresponding to a VP2 protein, 23.9 kDa corresponding to a VP3 protein, and 8.9 kDa corresponding to a VP4 protein, and it was visually confirmed that a high yield of proteins were secreted into the culture medium.

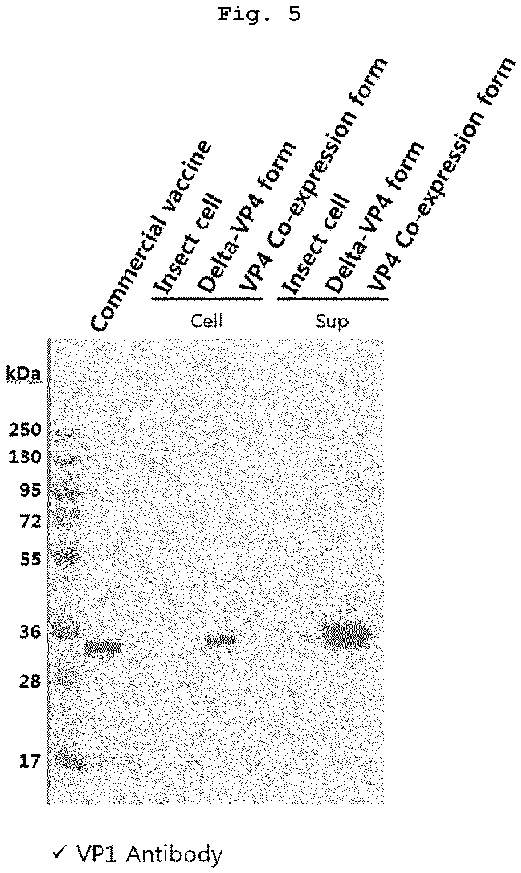

In addition, as shown in FIG. 5, as a result of performing Western blot using an antibody specific to the VP1 protein, it was clearly confirmed that the protein identified in SDS-PAGE was the VP1 protein. In addition, it was verified that VP1 was cleaved from P1 by the normal expression of 3C. In particular, high secretion efficiency was also confirmed in the Delta-VP4 form, but the highest level of structural protein expression was confirmed in the VP4 Co-expression form.

Additionally, the proteins expressed from the culture (cells and supernatant) of the recombinant virus transformed into the Delta-VP4 formm were analyzed.

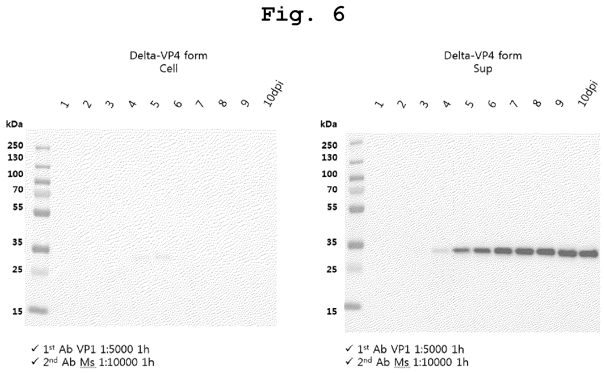

As a result, as shown in FIG. 6, it was confirmed that the secretion efficiency into the culture medium was high after expression even in the Delta-VP4 form, similarly to the VP4 Co-expression form.

Through the above results, it was determined that when VP4 was removed from the FMDV structural protein P1 (VP4-VP2-VP3-VP1) and expressed, as in the VP4 Co-expression form and the Delta-VP4 form, the protein was secreted into the culture medium after expression, and such secretion contributed significantly to the productivity of antigen candidates.

Meanwhile, the proteins expressed from a culture (cells and supernatant) of the recombinant virus transformed into the Full form were analyzed.

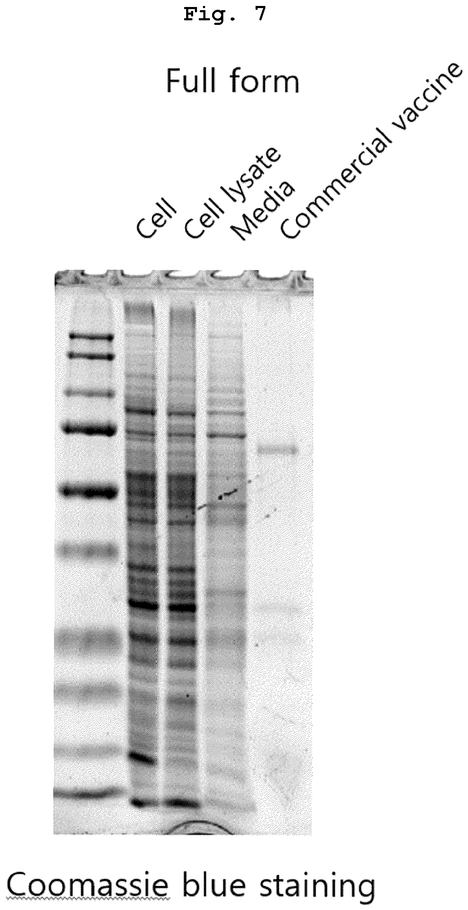

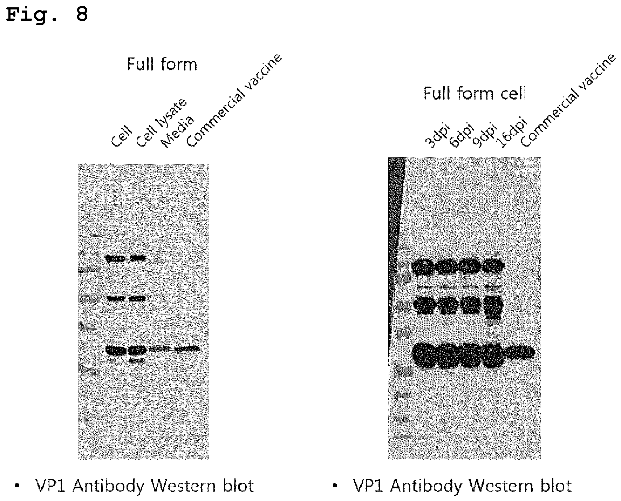

As a result, as shown in FIGS. 7 and 8, in the Full form, a large amount of protein was observed to be present in the cells after expression, but no secretion into the supernatant was observed even as dpi (days post inoculation) increased.

These results suggeste that the vector forms (Delta-VP4 form, VP4 Co-expression form), in which VP4 is removed from the FMDV structural protein P1 (VP4-VP2-VP3-VP1), play an important role in the production of antigen candidates.

### <2-2> Comparison of antigen amounts by performing ELISA using an antibody specific to FMDV VP1

ELISA was performed to compare the amounts of FMDV antigens produced from the recombinant virus of the present disclosure.

Specifically, the recombinant virus was inoculated into Hi5 insect cells, and after several days, the culture medium was collected by centrifugation at 4000 rpm for 5 minutes at 4°C. ELISA was performed using an antibody specific to FMDV VP1 with the collected culture medium sample. The commercial vaccine used as a control was the BIOAFTOGEN FMD vaccine.

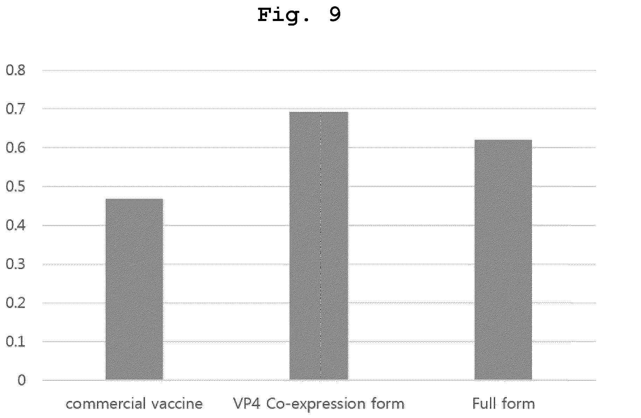

As a result, as shown in FIG. 9, a higher value was confirmed in the VP4 Co-expression form, and thus the VP4 Co-expression form was selected as the optimal expression form for FMDV structural proteins.

### <Example 3> Analysis of expressed proteins using antibodies specific to VP1, VP2, VP3, and VP4 structural proteins

An experiment was performed to confirm that all FMDV structural proteins were expressed by the recombinant virus.

Specifically, Western blot was performed using specific antibodies specific to VP0, VP1, VP2, VP3, and VP4 in a culture medium mass-cultured using the recombinant virus. The commercial vaccine used as a control group was BIOAFTOGEN FMD vaccine.

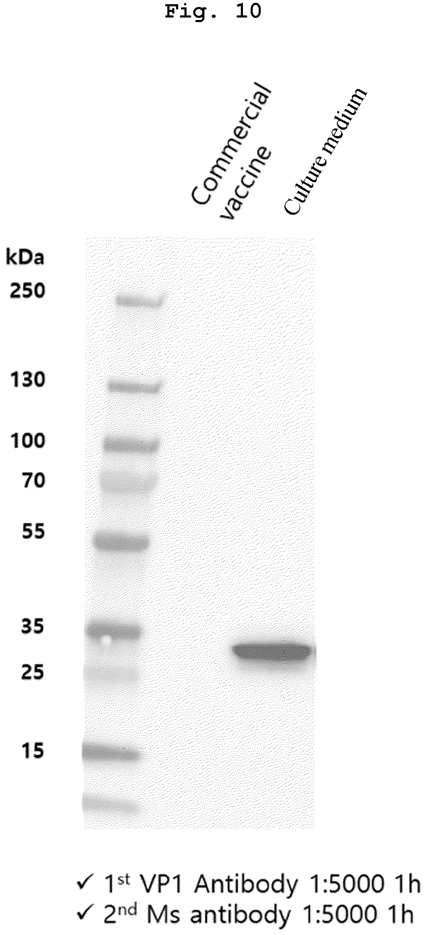

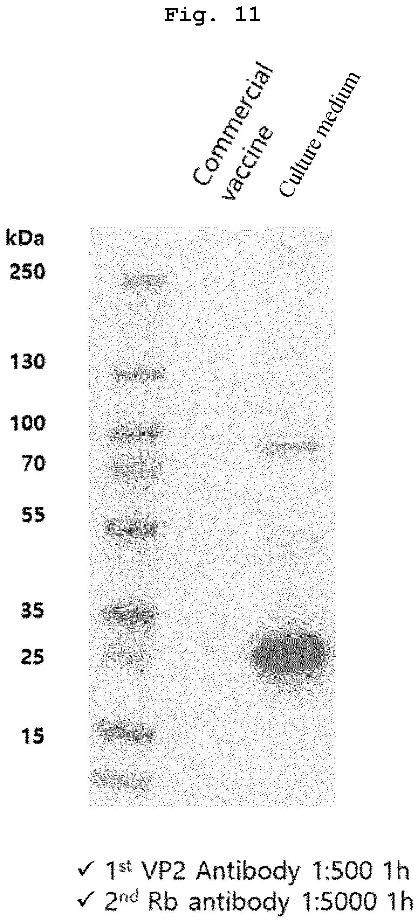

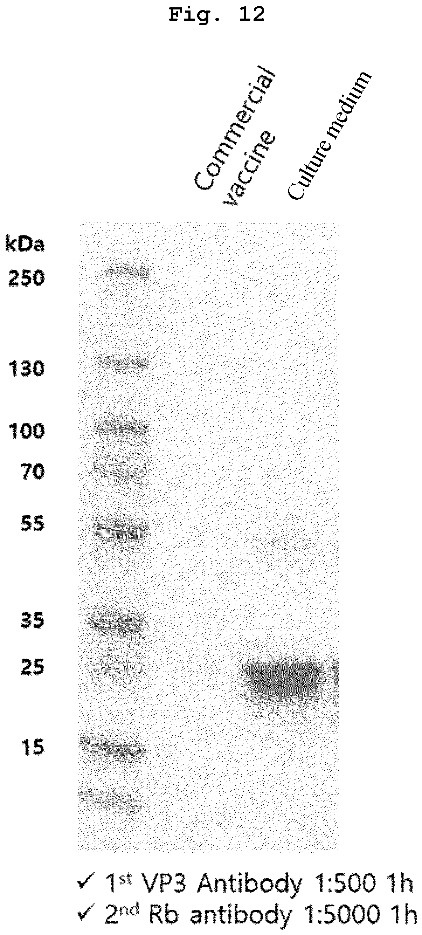

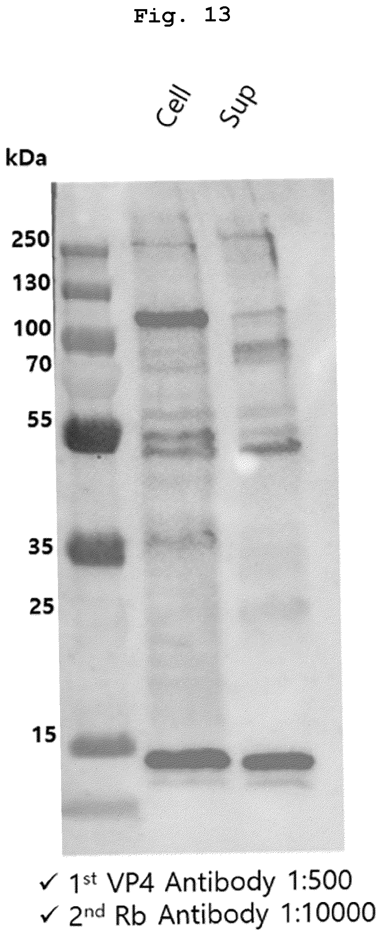

As a result, as shown in FIGS. 10 to 13, individual bands corresponding to the FMDV structural proteins VP1, VP2, VP3, and VP4 were confirmed for the recombinant virus of O type. These results confirmed that all proteins existed individually after expression, indicating that the 3C protease was expressed and functioned normally.

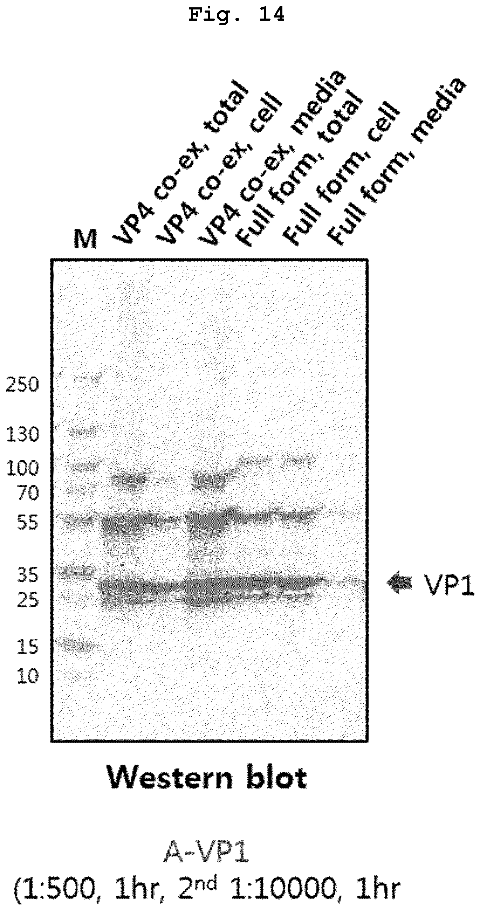

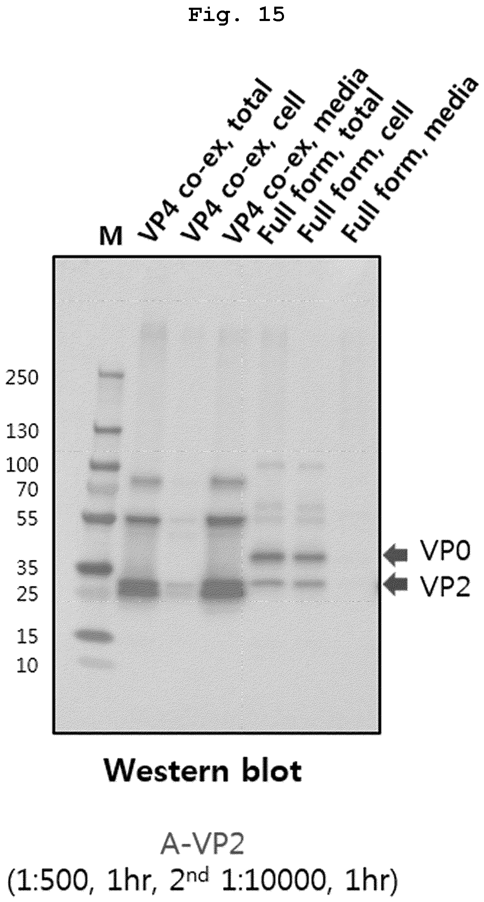

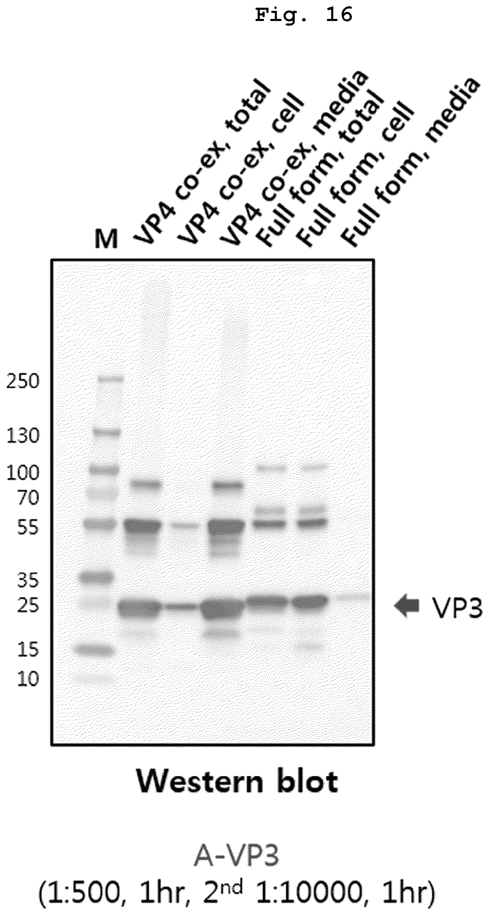

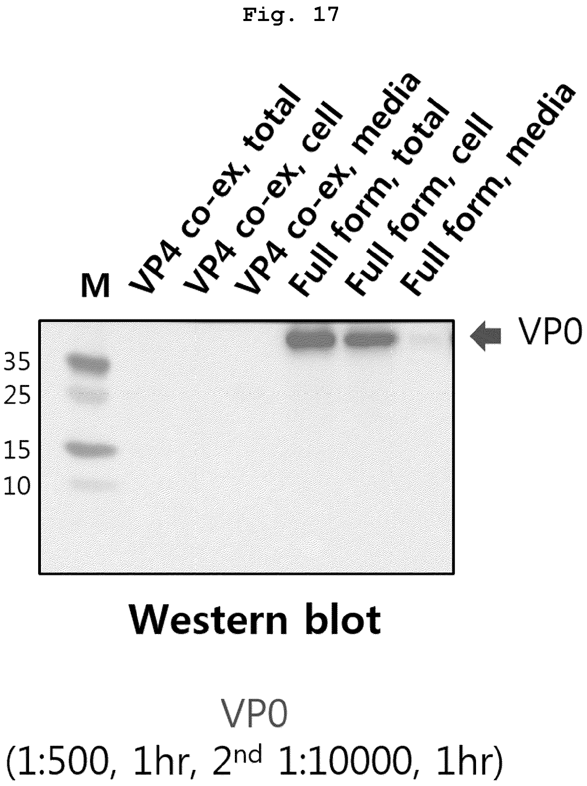

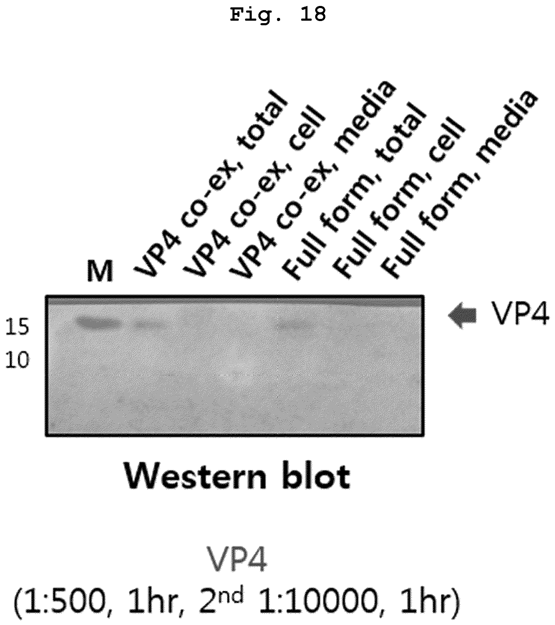

In addition, as shown in FIGS. 14 to 18, it was confirmed that the FMDV structural proteins VP1, VP2, VP3, and VP4 were all expressed by the recombinant virus for type A. In particular, in the VP4 Co-expression form, VP2 was expressed alone, and a band of approximately 24.4 kDa was confirmed. In contrast, in the Full form, VP0 was expressed as a bound form of VP2 and VP4 (FIG. 15). Furthermore, in the VP4 Co-expression form, VP0 was not detected, indicating that VP0 was cleaved into VP2 and VP4. However, in the Full form, VP0 was expressed as a bound form of VP2 and VP4 (FIG. 17).

As a result, unlike the full form, in the VP4 Co-expression form, each of the proteins VP1, VP2, VP3, and VP4 was expressed individually and secreted outside the cell, indicating that the 3C protease was expressed and functioned normally.

### <Example 4> Morphological observation of FMDV antigen candidates using TEM

An experiment was performed to analyze the characteristics of the proteins expressed in above Examples.

Specifically, the culture medium of the recombinant virus using the VP4 Co-expression form was concentrated and purified. Using a tangential flow filtration (TFF) system, the culture medium was concentrated 20-fold by passing through a 100 kDa hollow fiber filter and then exchanged with 20 mM Tris-HCl, pH 6.5 buffer. The obtained concentrate of the culture medium was subjected to IEX (Ion Exchange Chromatography) using Captocore Q Impress. The purified FMDV antigen candidates were confirmed by SDS-PAGE and Western blot.

To observe the morphology of the FMDV antigen candidates obtained through purification, transmission electron microscope (TEM) was performed.

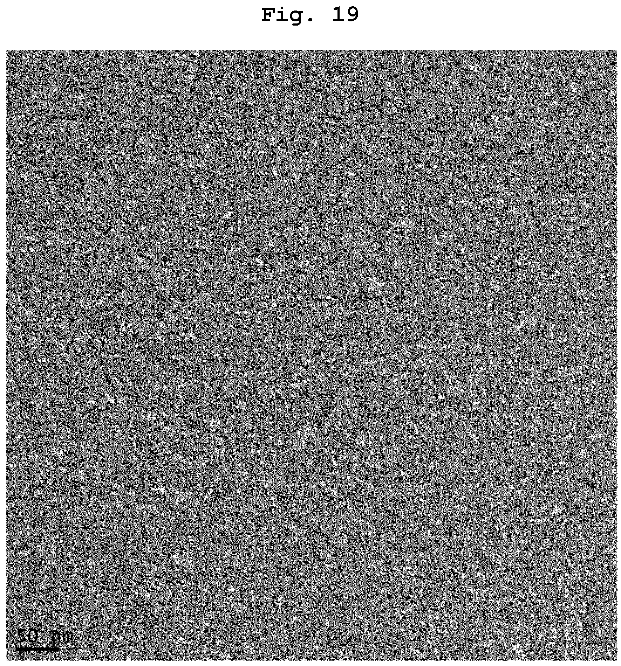

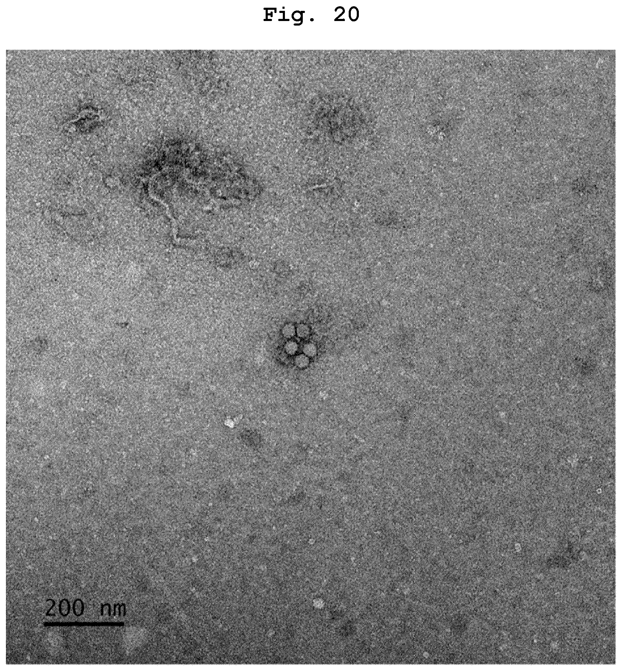

As a result, as shown in FIG. 19, it was confirmed that the nanoparticles had a size of approximately 10 to 15 nm. In addition, as shown in FIG. 20, it was confirmed that all VLP particles had an appropriate size of approximately 25 to 30 nm.

### <Example 5> Immunogenicity test of vaccine candidates

To confirm the immune efficacy of vaccine candidates, a vaccine was prepared for administration into a target animal.

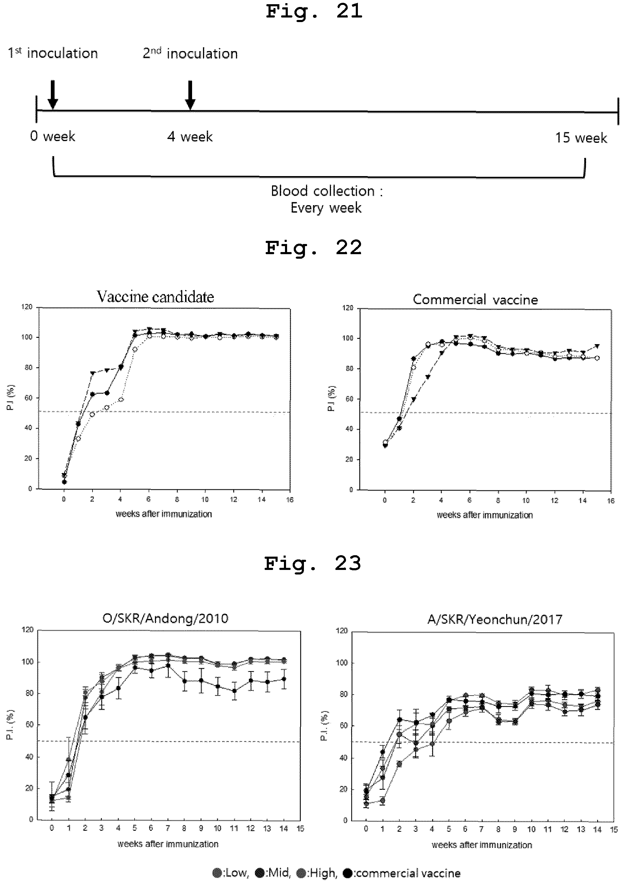

Specifically, the vaccine candidate used was composed of structural proteins for serotype O, produced from a recombinant virus constructed using the VP4 Co-expression form, and emulsified into an oil emulsion. The vaccine was administered twice, with the first dose given at week 0 of the test and the second dose given at week 4. Blood was collected weekly up to week 15, and porcine serum was isolated (FIG. 21). The antibody titer was measured using the isolated serum with an ELISA kit (PrioCHECK FMDV Type O Andibody SP ELISA kit). The commercial vaccine used as a control was the BIOAFTOGEN FMD vaccine.

As a result, as shown in FIG. 22, 100% percentage inhibition was confirmed in all subjects, and the immunogenicity was confirmed to be superior compared to the control group.

### <Example 6> Immunogenicity test according to vaccine candidate concentration

Antibody titers and neutralizing antibody titers (VNT) were evaluated according to a concentration of a vaccine candidate.

Specifically, the vaccine was administered twice, with the first dose at week 0 of the test and the second dose at week 4. Separate vaccine were prepared and applied for type O and type A. Serum was collected weekly from pigs inoculated with each serotype, and the antibody titer were measured by week using the collected serum and ELISA kits (PrioCHECK FMDV Type O Andibody SP ELISA kit, PrioCHECK FMDV Type A Andibody SP ELISA kit). The commercial vaccine used as a control was the BIOAFTOGEN FMD vaccine.

As a result, as shown in FIG. 23, the O type showed antibody titers equivalent to those of the control group at all antigen amounts. The A type achieved 50% of antibody titer 2 weeks later than the control group at low doses, and showed similar increases in all other doses.

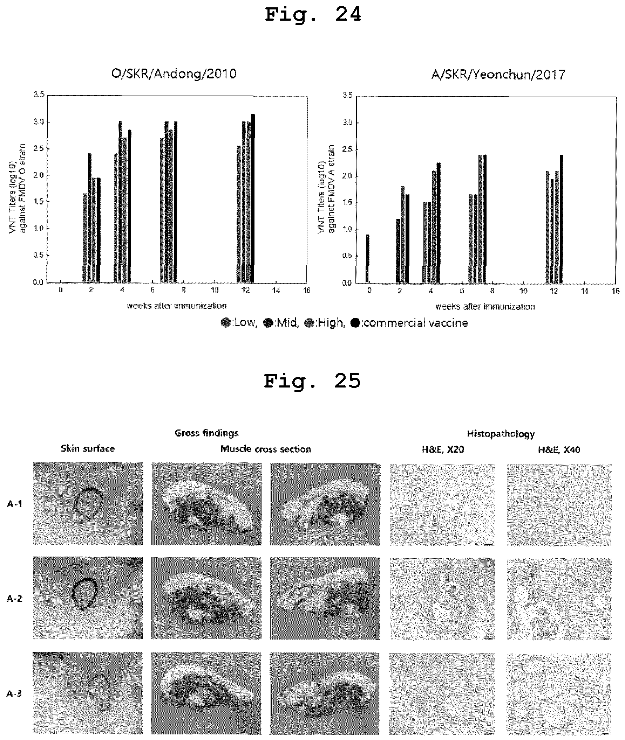

In addition, as shown in FIG. 24, when the neutralizing antibody titer (VNT) were measured, the O type reached a 32-fold titer from week 2 at all doses and was maintained at over 100-fold thereafter. In the A type, neutralizing antibodies formed later at the low dose; at the medium dose, they began increasing from week 2 and continued to rise gradually; and at the high dose, a 32-fold titer was achieved from week 2.

### <Example 7> Confirmation of safety for vaccine candidate administeration group

The skin of pigs vaccinated with a vaccine candidate was observed histopathologically.

Specifically, the vaccine candidate used was composed of structural proteins for serotype O, and was produced from a recombinant virus prepared using the VP4 Co-expression form. The vaccine was administered twice, the first dose was given at week 0 of the test, and the second dose at week 4. The injection site was then observed. The commercial vaccine used as a control group was the BIOAFTOGEN FMD vaccine.

**[Table 1]**

| Animal No. | Gross Finding* | | Lesion area | |
|---|---|---|---|---|
| | Skin surface | Muscle crose section | Length X Width (c m) | Thickness (cm) |
| A-1 | NM | NM | 0.0 X 0.0 | 0.0 |
| A-2 | NM | Firm and discoloration lesions (Granulomatous nodule formation | 2.5 X 1.0 | 1.0 |
| A-3 | NM | Focal discoloration lesions | 2.0 X 1.0 | 1.0 |
| G-1 | NM | Firm and discoloration lesions (Granulomatous nodule formation) | 6.5 X 6.5 | 2.5 |
| G-2 | NM | Firm and discoloration lesions (Granulomatous nodule formation) | 6.0 X 3.5 | 2.0 |
| G-3 | NM | Firm and discoloration lesions (Granulomatous nodule formation) | 5.5 X 3.5 | 2.0 |

| | | | | |
|---|---|---|---|---|
| * NM: No remarkable gross lesions. | | | | |

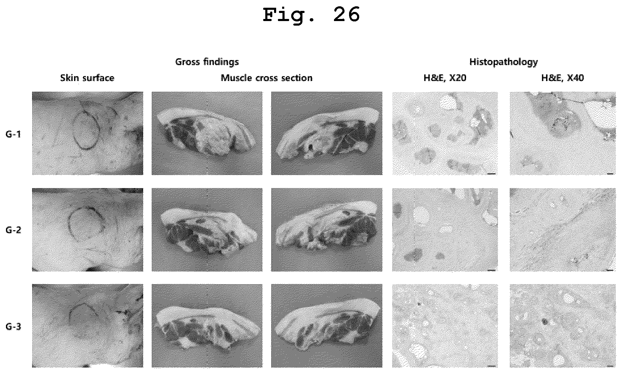

As a result, as shown in Table 1, and FIGS. 25 and 26, it was confirmed that the overall degree of lesion formation was low in the case of the vaccine candidate using the VP4 Co-exression form. Meanwhile, in the control group, macroscopic lesions were significantly observed, severe chronic granulomatous inflammation was confirmed, and extensive muscle cell degeneration and necrosis were found to be in progress.

The results indicate that the vaccine candidate of the present disclosure has a low side effect of abnormal meat occurrence.

## Claims

1. A recombinant expression vector comprising a first promoter and genes encoding Foot-and-Mouth Disease Virus (FMDV) proteins VP2, VP3, VP1 and 3C operably linked to the promoter.

2. The recombinant expression vector of claim 1, further comprising:
a second promoter and a gene encoding an FMDV protein VP4 operably linked to the promoter.

3. The recombinant expression vector of claim 1, wherein the first promoter and the second promoter are arranged in opposite directions within the vector.

4. The recombinant expression vector of claim 1, wherein the recombinant vector is expressed by a baculovirus vector.

5. The recombinant expression vector of claim 1, wherein the FMDV is selected from the group consisting of serotypes O, A, Asia1, C, SAT1, SAT2, and SAT3.

6. A transformant transformed with the recombinant expression vector according to claim 1 or 2.

7. A virus-like particle (VLP) or nanoparticle (NP) produced by the transformant according to claim 6.

8. A method for preparing virus-like particles or nanoparticles for preventing diseases caused by FMDV infection, the method comprising:
(1) preparing a recombinant expression vector according to claim 1 or 2;
(2) preparing a transformant using the recombinant expression vector;
(3) transfecting the transformant into a host cell;
(4) culturing the transfected host cell and collecting a culture medium; and
(5) isolating virus-like particles or nanoparticles from the culture medium.

9. A vaccine composition for a FMDV, comprising the virus-like particles or nanoparticles according to claim 7 as an active ingredient.

10. The vaccine composition of claim 9, further comprising:
an adjuvant.

11. The vaccine composition of claim 10, wherein the adjuvant is an oil emulsion.

12. A method for preventing diseases caused by FMDV infection, comprising administering the vaccine composition according to claim 9.

13. A use of the virus-like particles or nanoparticles according to claim 7 for the prevention of FMDV diseases.
